(19) Europäisches Patentamt / European Patent Office / Office européen des brevets

(11) **EP 3 727 678 B1**

(12) **FASCICULE DE BREVET EUROPEEN**

(45) Date de publication et mention de la délivrance du brevet:
**15.02.2023 Bulletin 2023/07**

(21) Numéro de dépôt: **18833283.7**

(22) Date de dépôt: **17.12.2018**

(51) Classification Internationale des Brevets (IPC):
**B01J 20/18** (1980.01)    **B01J 20/28** (1980.01)
**B01J 20/30** (1980.01)    **C07C 7/13** (1974.07)

(52) Classification Coopérative des Brevets (CPC):
(C-Sets disponibles)
**B01J 20/18; B01J 20/2803; B01J 20/3071;**
**B01J 20/3078; B01J 20/3085; C07C 7/13**    (Cont.)

(86) Numéro de dépôt international:
**PCT/FR2018/053327**

(87) Numéro de publication internationale:
**WO 2019/122649 (27.06.2019 Gazette 2019/26)**

(54) **ADSORBANTS ZÉOLITIQUES CONTENANT DU STRONTIUM ET DU BARYUM**

STRONTIUM UND BARIUM ENTHALTENDES ZEOLITHISCHES ADSORPTIONSMITTEL

ZEOLITIC ADSORBENTS CONTAINING STRONTIUM AND BARIUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2017 FR 1763036**

(43) Date de publication de la demande:
**28.10.2020 Bulletin 2020/44**

(73) Titulaires:
• **ARKEMA FRANCE**
  **92700 Colombes (FR)**
• **IFP Energies nouvelles**
  **92500 Rueil-Malmaison (FR)**

(72) Inventeurs:
• **BOUVIER, Ludivine**
  **64300 ORTHEZ (FR)**
• **PEREZ-PELLITERO, Javier**
  **69007 LYON (FR)**
• **LABEDE, Marie-Laurence**
  **64230 LESCAR (FR)**
• **BLANCKE, Guillaume**
  **69340 FRANCHEVILLE (FR)**

(74) Mandataire: **Bandpay & Greuter**
**30, rue Notre-Dame des Victoires**
**75002 Paris (FR)**

(56) Documents cités:
**WO-A1-2014/090771    FR-A1- 2 329 623**
**US-A- 4 957 514    US-A- 5 177 299**

• **Douglas M Ruthven ET AL: "REVIEW ARTICLE NUMBER 31 COUNTER-CURRENT AND SIMULATED COUNTER-CURRENT ADSORPTION SEPARATION PROCESSES", Chemical Engineering Science, 1 janvier 1989 (1989-01-01), pages 101-1038, XP055501110, Extrait de l'Internet: URL:https://ac.els-cdn.com/0009250989870022/1-s2.0-0009250989870022-main.pdf?_tid=9ba50ed2-29da-4965-8d74-2cac577a9851&acdnat=1534858728_81aff691e6254376206f6e30d59c1336**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 727 678 B1

(52) Classification Coopérative des Brevets (CPC):
(Cont.)

C-Sets
**C07C 7/13, C07C 15/08**

**Description**

L'invention concerne des adsorbants à base de cristaux agglomérés de zéolithe X comprenant du baryum et du strontium.

Ces adsorbants peuvent être utilisés plus particulièrement pour la production en phase liquide ou phase gaz de para-xylène très pur à partir d'une charge d'hydrocarbures aromatiques contenant des isomères à 8 atomes de carbone.

Le marché du paraxylène de haute pureté est considéré comme un marché en forte expansion, ses débouchés étant principalement la production d'acide téréphtalique (PTA) obtenu par oxydation du paraxylène, à l'origine des fibres polyesters utilisées pour les vêtements et les résines et films de polyéthylène-téréphtalate (PET).

Le *para*-xylène de haute pureté est aujourd'hui le plus souvent obtenu produit par valorisation des xylènes selon un procédé dit de « boucle C8-aromatiques », incluant des étapes de séparation (élimination des composés lourds dans la « colonne des xylènes », extraction du paraxylène) et d'isomérisation des xylènes. L'extraction du paraxylène de haute pureté par adsorption sélective est par ailleurs bien connue de l'art antérieur.

L'arrière-plan technologique décrivant la production de paraxylène à très haute pureté est illustré dans le brevet FR2681066 (Institut Français du Pétrole), et se base sur la séparation du paraxylène à partir d'une charge d'hydrocarbures aromatiques essentiellement à 8 atomes de carbone au sein d'un adsorbeur par contact avec un lit d'adsorbant zéolithique en présence d'un solvant de désorption (désorbant) approprié.

Il est également connu dans l'art antérieur que des adsorbants zéolithiques comprenant au moins de la zéolithe Faujasite (FAU) de type X ou Y et comprenant, outre des cations sodium, des ions baryum, potassium ou strontium, seuls ou en mélanges, sont efficaces pour adsorber sélectivement le paraxylène dans un mélange d'hydrocarbures aromatiques.

Les brevets US3558732 et US4255607 montrent que des adsorbants zéolithiques comprenant des aluminosilicates à base de potassium ou à base de baryum, ou encore à base de potassium et de baryum, sont efficaces pour la séparation du paraxylène présent dans des coupes aromatiques en C8 (coupes comprenant des hydrocarbures aromatiques à 8 atomes de carbone).

FR2329623 divulgue un adsorbant comprenant une zéolite de type X ou Y contenant du baryum et du strontium sur les sites cationiques échangeables. L'adsorbant est utilisé dans un procédé de séparation du para-xylène et du para-diéthylbenzène.

Le brevet US3997620 présente des agglomérés, dans lesquels le liant d'agglomération n'est pas zéolithisé, lesdits agglomérés étant échangés au baryum et au strontium, de sorte que le ratio pondéral Ba/Sr est compris entre 1:1 et 15:1.

Plus particulièrement, ce document enseigne que des adsorbants à base de zéolithes de type X ou de type Y et comprenant du baryum et du strontium, en leurs sites cationiques échangeables, avec un ratio pondéral baryum/strontium d'environ 1:1 à environ 15:1, peuvent être avantageusement utilisés dans un procédé de récupération de *para*-xylène. Avec ce ratio, l'avantage observé est une sélectivité améliorée du *para*-xylène par rapport au désorbant, le *para*-diéthylbenzène, mais aussi une sélectivité améliorée du *para*-xylène par rapport aux autres isomères du xylène.

Hormis ce document montrant un certain avantage, eu égard à la sélectivité, pour des agglomérés contenant des proportions importantes de strontium, il n'en reste pas moins que dans la littérature scientifique, comme dans la littérature brevets, les documents enseignent principalement que les zéolithes comprenant des cations baryum ou des cations baryum et potassium se montrent particulièrement efficaces en termes de sélectivité pour la séparation d'isomères d'hydrocarbures aromatiques à 8 atomes de carbone. Les zéolithes industrielles utilisées dans ces applications de séparation d'isomères sont conventionnellement préparées à partir de zéolithes comprenant du sodium en tant que cation compensateur, puis les ions sodium sont échangés par des ions baryum ou des ions baryum et potassium.

Ces opérations d'échanges sont habituellement réalisées à partir de solution aqueuse d'halogénure de baryum, et en particulier de chlorure de baryum ($BaCl_2$). Cependant, les coûts du chlorure de baryum de très haute pureté, comme requis pour la synthèse desdites zéolithes, étant élevés, l'échange avec ce sel très pur se révèle de moins en moins compétitif pour des préparations de zéolithes à grande échelle.

En effet, le chlorure de baryum est le plus souvent obtenu à partir de minerais, tels que par exemple la barytine ou la benstonite, ces minerais comprennent généralement d'autres métaux alcalin ou alcalino-terreux dont du strontium, en particulier. Par conséquent, les différentes sources d'ions baryum, par exemple sous forme de chlorure de baryum, disponibles commercialement, et lorsqu'elles ne sont pas de très grande pureté, peuvent contenir des quantités non négligeables d'impuretés sous forme de sels de strontium, par exemple de l'ordre de 500 ppm poids à quelques pourcents en poids de chlorure de strontium.

Les coûts des sources de baryum sous forme de chlorure de baryum de très grande pureté ainsi que l'impact environnemental des opérations de purification du chlorure de baryum conduisent à trouver des alternatives qui s'affranchissent de ces problèmes, tout en permettant de conserver une efficacité maximale, notamment en termes de sélectivité et de productivité, lorsque les agglomérés échangés au baryum sont utilisés pour la récupération de para-xylène dans des coupes aromatiques en C8.

En outre, il est toujours intéressant de pouvoir disposer de plusieurs fournisseurs de sources de baryum, en particulier de chlorure de baryum, tout en pouvant s'affranchir de conditions drastiques de pureté, et de disposer de différentes sources, certaines pouvant s'avérer moins « pures » en termes de teneurs en baryum, que d'autres qui contiennent des quantités variables en chlorure de strontium.

Il a maintenant été découvert que les objectifs précités peuvent être atteints, en totalité ou au moins en partie, grâce à la présente invention qui est maintenant exposée dans la description qui suit. D'autres objectifs encore pourront apparaître dans la description détaillée ci-dessous.

Ainsi, et selon un premier aspect, la présente invention concerne un adsorbant à base de cristaux de zéolithe(s) agglomérés, ledit aggloméré comprenant :

- au moins des cristaux de zéolithe(s) FAU, de ratio molaire Si/Al compris entre 1,00 et 1,50, bornes incluses,
- une teneur pondérale en ions baryum ($Ba^{2+}$), exprimée en poids d'oxyde de baryum (BaO), strictement supérieure à 30%, de préférence strictement supérieure à 33%, de préférence encore une teneur comprise entre 34% et 42%, bornes incluses, et de manière tout à fait préférée comprise entre 34% et 40%, bornes incluses, par rapport au poids total de l'adsorbant,
- une teneur pondérale en ions strontium ($Sr^{2+}$), exprimée en poids d'oxyde de strontium (SrO), strictement supérieure à 0,1% et strictement inférieure à 3%, de préférence comprise entre 0,15% et 2,9%, bornes incluses, de préférence encore comprise entre 0,15% et 2,5% et de manière tout à fait préférée comprise entre 0,15% et 2,4%, bornes incluses, par rapport au poids total de l'adsorbant; et dans lequel l'adsorbant présente un ratio pondéral baryum/strontium compris entre 16:1 et 400:1.

Dans la présente invention, il doit être compris que les teneurs pondérales exprimées en poids d'oxydes sont exprimées par rapport au poids total de l'adsorbant anhydre (poids corrigé de la perte au feu).

Les adsorbants selon l'invention peuvent également comprendre une phase non zéolithique, c'est-à-dire une phase non cristalline qui est essentiellement inerte vis-à-vis de l'adsorption, comme expliqué plus loin. Dans le cas où l'adsorbant selon l'invention comprend une telle phase non zéolithique, les teneurs en oxydes définies plus haut prennent compte des oxydes compris dans ladite phase non zéolithique.

L'adsorbant selon la présente invention est un adsorbant à base de cristaux de zéolithe(s) FAU de type X. Par « zéolithe FAU de type X», on entend les zéolithes dont le ratio atomique Si/Al est compris entre 1,00 et 1,50 bornes incluses, de préférence entre 1,00 et 1,45, de préférence encore entre 1,05 et 1,45, bornes incluses et de manière encore plus préférée entre 1,10 et 1,45 bornes incluses.

Parmi les zéolithes X, il est maintenant communément admis de reconnaître deux sous-groupes dénommés zéolithes LSX et zéolithes MSX. Les zéolithes LSX présentent un ratio atomique Si/Al égal à environ 1 et les zéolithes MSX présentent un ratio atomique Si/Al compris entre environ 1,05 et environ 1,15, bornes incluses.

La définition de zéolithe FAU comprend également les zéolithes FAU de type X définies ci-dessus à porosité hiérarchisée, c'est-à-dire les zéolithes de type X à porosité hiérarchisée (ou zéolithe XPH), les zéolithes de type MSX à porosité hiérarchisée (ou MSXPH) et les zéolithes de type LSX à porosité hiérarchisée (ou LSXPH), et plus particulièrement les zéolithes FAU à porosité hiérarchisée et de rapport atomique Si/Al compris entre 1,00 et 1,50, bornes incluses, de préférence entre 1,05 et 1,50, de préférence encore entre 1,05 et 1,40, bornes incluses, et de manière encore plus préférée, entre 1,15 et 1,40, bornes incluses.

Par « zéolithe à porosité hiérarchisée », on entend une zéolithe possédant à la fois des micropores et des mésopores, autrement dit une zéolithe à la fois microporeuse et mésoporeuse. Par « zéolithe mésoporeuse », on entend une zéolithe dont les cristaux zéolithiques microporeux présentent, conjointement à la microporosité, des cavités internes de taille nanométrique (mésoporosité), facilement identifiables par observation au moyen d'un Microscope Électronique à Transmission (MET ou « TEM » en langue anglaise), comme décrit par exemple dans US7785563 : l'observation par microscopie électronique à transmission (MET) permet de vérifier si les cristaux zéolithiques sont des cristaux de zéolithe pleins (i.e. non mésoporeux) ou des agrégats de cristaux de zéolithes pleins ou des cristaux mésoporeux ou des agrégats de cristaux mésoporeux.

L'adsorbant selon l'invention englobe également les adsorbants comprenant des mélanges de deux ou plusieurs zéolithes FAU telles qu'elles viennent d'être définies, mais aussi comprenant des mélanges d'une ou plusieurs zéolithes FAU avec une ou plusieurs autres zéolithes bien connue(s) de l'homme du métier. On préfère toutefois les adsorbants à base de cristaux de zéolithe FAU de type X, comme défini ci-dessus.

La structure des cristaux de zéolithe(s) dans l'adsorbant de la présente invention est aisément identifiable par toute méthode bien connue de l'homme du métier et en particulier par Diffraction des Rayons X (également dénommée « analyse par DRX »).

Dans le présent document, on emploie l'appellation « diamètre moyen en nombre » ou bien « taille » pour les cristaux de zéolithe(s) et pour l'adsorbant selon l'invention. La méthode de mesure de ces grandeurs est explicitée plus loin dans la description. Selon un mode de réalisation préféré de la présente invention, le diamètre moyen en nombre des cristaux de zéolithe(s) est inférieur ou égal à 1,5 $\mu$m, de préférence compris entre 0,1 $\mu$m et 1,2 $\mu$m, de manière plus préférée compris entre 0,1 $\mu$m et 1,0 $\mu$m, bornes incluses.

Selon un mode de réalisation préféré, la teneur pondérale totale en ions alcalins ou alcalino-terreux autres que $Ba^{2+}$ et $Sr^{2+}$, exprimée en poids d'oxydes d'alcalins ou d'alcalino-terreux respectivement, est inférieure à 5% et de préférence est comprise entre 0% et 2% et avantageusement entre 0% et 1%, bornes incluses, par rapport au poids total de l'adsorbant.

On préfère en outre dans le cadre de la présente invention les adsorbants comprenant une teneur pondérale en ions sodium ($Na^+$), exprimée en poids d'oxyde de sodium ($Na_2O$), strictement inférieure à 0,3%, de préférence strictement inférieure à 0,2%, par rapport au poids total de l'adsorbant.

Selon encore un autre mode de réalisation préféré de l'invention, l'adsorbant comprend une teneur en ions potassium ($K^+$), exprimée en poids d'oxyde de potassium ($K_2O$), inférieure à 9%, de préférence inférieure à 8%, mieux encore inférieure à 5%, et de préférence encore comprise entre 0% et 2%, avantageusement entre 0% et 1%, bornes incluses, par rapport au poids total de l'adsorbant.

De préférence, la teneur pondérale totale en ions alcalins ou alcalino-terreux autres que $Ba^{2+}$, K+ et $Sr^{2+}$, exprimée en poids d'oxydes d'alcalins ou d'alcalino-terreux respectivement, est inférieure à 5% et de préférence est comprise entre 0% et 2% et avantageusement entre 0% et 1%, bornes incluses, par rapport au poids total de l'adsorbant.

Selon un mode de réalisation de la présente invention, le ratio pondéral baryum/strontium dans l'adsorbant est compris entre 16:1 et 400:1, de préférence compris entre 16:1 et 300:1, de préférence encore compris entre 16:1 et 200:1.

L'adsorbant de la présente invention est de préférence sous la forme d'agglomérats, c'est-à-dire qu'il est constitué de cristaux de zéolithe(s) et d'au moins une phase non zéolithique comprenant au moins un liant d'agglomération permettant la cohésion des cristaux entre eux.

Le liant d'agglomération peut être zéolithisable. Il contient alors au moins 80 % de préférence, au moins 90%, de préférence encore au moins 95%, plus particulièrement au moins 96%, en poids, d'argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique (c'est-à-dire matière active au sens de l'adsorption), le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

D'autres argiles telles que notamment la sépiolite ou l'attapulgite peuvent également être utilisées. Dans tous les cas, les argiles peuvent être utilisées dans leur état brut ou peuvent être préalablement soumises à un ou plusieurs traitements, par exemple choisis parmi calcination, traitement à l'acide, modification chimique, et autres.

Ainsi, l'adsorbant selon la présente invention comprend des cristaux de zéolithe(s), et au moins une phase non zéolithique (PNZ), c'est-à-dire une phase non cristalline qui est essentiellement inerte vis-à-vis de l'adsorption. Le taux de cristallinité de l'adsorbant selon l'invention est mesuré par analyse par diffraction de rayons X, connue de l'homme du métier sous l'acronyme DRX, comme indiqué plus loin. Il est, de manière générale, supérieur à 80%, c'est-à-dire que la PNZ est inférieure à 20%.

Dans un mode de réalisation préféré de la présente invention, la teneur pondérale en phase non zéolithique (PNZ) est inférieure à 15%, de préférence inférieure à 10%, de préférence encore inférieure à 5%. Selon un aspect tout à fait préféré, la PNZ de l'adsorbant est comprise entre 0 et 15% de préférence comprise entre 0 et 10%, de préférence encore comprise entre 0 et 5% bornes non comprises, de manière tout à fait préférée comprise entre 0,1% et 5%, mieux encore entre 0,5% et 5% et de manière tout particulièrement préférée entre 2% et 5%, bornes non comprises, par rapport au poids total de l'adsorbant anhydre (poids corrigé de la perte au feu).

Le diamètre moyen en nombre de l'adsorbant selon l'invention est avantageusement et le plus souvent compris entre 0,2 mm et 2 mm, plus particulièrement entre 0,2 mm et 0,8 mm et de préférence entre 0,2 mm et 0,65 mm, bornes incluses.

Selon un mode de réalisation préféré, l'adsorbant selon l'invention présente une perte au feu mesurée à 950 °C selon la norme NF EN 196-2 comprise entre 4,0% et 7,7%, préférence entre 4,5 et 6,5 % et avantageusement entre 4,8 et 6%, bornes incluses.

L'adsorbant selon la présente invention présente préférentiellement une résistance mécanique généralement supérieure ou égale à 1,8 MPa, typiquement supérieure ou égale à 2,1 MPa. Cette résistance mécanique est mesurée par la méthode Shell série SMS1471-74 adaptée pour des agglomérés de taille inférieure à 1,6 mm.

La capacité d'adsorption de l'adsorbant selon l'invention est quant à elle mesurée par mesure du volume microporeux de l'adsorbant évalué d'après l'équation de Dubinin-Raduskevitch par adsorption d'azote ($N_2$) à une température de 77K, après dégazage sous vide à 300°C pendant 16 heures. Le volume microporeux de l'adsorbant de l'invention est ainsi mesuré comme étant le plus souvent strictement supérieur à 0,245 $cm^3.g^{-1}$, de préférence strictement supérieur à 0,250 $cm^3.g^{-1}$, de préférence encore compris entre 0,250 $cm^3.g^{-1}$ et 0,300 $cm^3.g^{-1}$, bornes non comprises.

L'adsorbant selon la présente invention, qui comprend avantageusement une teneur réduite, voire très réduite, en phase non zéolithique, c'est-à-dire essentiellement inerte vis-à-vis de l'adsorption, est un adsorbant tout à fait performant pour la séparation des xylènes et dont les coûts de fabrication sont réduits par rapport aux adsorbants équivalents en termes d'adsorption et connus de l'art antérieur.

La réduction des coûts de fabrication de ces adsorbants est principalement obtenue grâce à l'utilisation d'un sel de baryum de pureté moindre que celle habituellement utilisée pour réaliser l'échange cationique.

L'adsorbant selon l'invention peut en effet être obtenu selon toute technique bien connue de l'homme du métier, et par exemple comme décrit dans la demande WO2014090771. Les procédés de synthèse d'adsorbants, et en particulier ceux destinés à la séparation des xylènes, comprend généralement une étape d'agglomération de cristaux de zéolithe(s) avec un liant, le plus souvent une argile, une étape de mise en forme, puis séchage et calcination, et enfin une étape d'échange cationique par mise en contact de l'aggloméré avec une solution d'ions alcalins et/ou alcalino-terreux.

Dans des modes de réalisation préférés, l'aggloméré, avant et/ou après échange cationique, est soumis à une ou plusieurs étapes de zéolithisation bien connue de l'homme du métier, comme décrit par exemple dans WO2014090771, c'est-à-dire de transformation de la totalité ou au moins une partie du liant d'agglomération en fraction cristalline zéolithique, active au sens de l'adsorption.

Dans le cas de la séparation des xylènes, l'échange cationique optimal reconnu dans ce domaine technique consiste à échanger la totalité ou au moins la plus grande partie des ions sodium présents dans les cristaux de zéolithe(s) de départ, par des ions baryum ou des ions baryum et des ions potassium. Cet échange ionique peut être effectué indifféremment sur les cristaux de zéolithe(s) ou sur les agglomérats de cristaux de zéolithe(s) avec le liant d'agglomération, avant et/ou après zéolithisation éventuelle, de préférence après zéolithisation, dudit liant.

Les étapes d'échange des cations des fractions zéolithiques s'effectuent selon les méthodes classiques connues de l'homme du métier, et le plus souvent par mise en contact des cristaux de zéolithe(s), agglomérés ou non avec un liant, avant et/ou après zéolithisation éventuelle, de préférence après zéolithisation, dudit liant, avec un sel du cation à échanger, tel que le chlorure de baryum ($BaCl_2$) pour échange au baryum et/ou chlorure de potassium (KCl) pour échange au potassium, en solution, le plus souvent en solution aqueuse, à une température comprise entre la température ambiante et 100°C, et de préférence comprise entre 80°C et 100°C.

Pour obtenir rapidement des teneurs en oxyde de sodium faibles, comme décrit précédemment, on préfère opérer avec un large excès d'ions baryum et/ou potassium par rapport aux cations de la zéolithe que l'on souhaite échanger, typiquement un excès de l'ordre de 10 à 12, avantageusement en procédant par échanges successifs.

Comme indiqué précédemment, cet échange cationique est le plus souvent réalisé avec une solution aqueuse, organique ou hydro-organique, de préférence une solution aqueuse, comprenant des ions baryum. Les solutions d'ions baryum utilisées présentent généralement une concentration en ions $Ba^{2+}$ pouvant varier entre 0,2 M et 2 M.

Il a maintenant été découvert de manière tout à fait surprenante que des solutions d'ions baryum pouvant contenir un taux d'impuretés, sous forme de sels strontium, pouvant aller jusqu'à 4% en poids, permettent d'obtenir des adsorbants présentant des performances d'adsorption, notamment dans l'application de séparation des xylènes, tout à fait comparables à celles des adsorbants connus de l'art antérieur et pour lesquels les échanges cationiques sont réalisés avec des solutions d'ions baryum de beaucoup plus grande pureté et donc plus onéreuses.

Plus spécifiquement, il a été observé qu'il est possible d'utiliser des sels de baryum, en particulier du chlorure de baryum, notamment des solutions aqueuses de chlorure de baryum, pouvant contenir jusqu'à 4% en poids d'impuretés sous forme de sel de strontium, de préférence entre 0,2% et 3% en poids, de manière préférée entre 0,2% et 2,5% en poids, bornes incluses par rapport au poids total du sel de baryum considéré.

L'impureté sous forme de sel de strontium est le plus souvent le chlorure de strontium ($SrCl_2$).

Selon un autre aspect, l'utilisation d'un sel de baryum comprenant du strontium dans les proportions indiquées ci-dessus, conduit à un adsorbant selon l'invention pour lequel le taux d'échange strontium, est au plus égal à 11%, de préférence au plus égal à 10%, de préférence encore au plus égal à 9% et de manière tout particulièrement préférée compris entre 0,5% et 8%. Ce taux d'échange correspondant au rapport molaire de l'oxyde de strontium sur la somme des oxydes de baryum, strontium, potassium et sodium.

Ainsi, la présente invention propose l'utilisation de sels de baryum dont les spécifications en termes de pureté sont abaissées, tout en permettant le maintien des propriétés de l'adsorbant échangé baryum, dans l'application considéré.

Il est donc possible grâce à la présente invention de disposer d'adsorbants performants pouvant contenir du strontium, dont la teneur, exprimée en oxyde (SrO) est strictement inférieure à 3% poids par rapport au poids total de l'adsorbant, ce qui correspond à un taux d'échange en strontium d'environ 11% au maximum et à une teneur en impuretés (sel de strontium) à environ 3% en poids dans la solution de baryum utilisée pour l'échange ionique.

Ces adsorbants peuvent être utilisés plus particulièrement pour la production en phase liquide ou phase gaz de *para*-xylène très pur à partir d'une charge d'hydrocarbures aromatiques contenant des isomères à 8 atomes de carbone.

Ainsi, et selon un autre aspect, la présente invention propose un procédé de séparation des xylènes mettant en oeuvre un adsorbant tel que décrit précédemment, permettant la production de *para*-xylène à haute pureté avec une productivité optimisée à partir d'une charge d'hydrocarbures aromatiques contenant des isomères à 8 atomes de carbone.

Plus particulièrement, l'invention concerne un procédé de récupération de para-xylène à haute pureté à partir de coupes d'isomères aromatiques à 8 atomes de carbone consistant à utiliser, un adsorbant selon l'invention, mis en oeuvre dans des procédés en phase liquide mais aussi en phase gazeuse comme agent d'adsorption du para-xylène en présence d'un désorbant, de préférence choisi parmi le toluène et le *para*-diéthylbenzène.

Par *para*-xylène de haute pureté, on entend un produit approprié pour une utilisation dans la production d'acide téréphtalique ou de téréphtalate de diméthyle, c'est à dire une pureté d'au moins 99,5% en poids, de préférence au moins 99,7% en poids, de préférence au moins 99,8% en poids et plus préférablement encore au moins 99,9% en poids. La pureté du *para*-xylène peut-être déterminée par des méthodes chromatographiques. Une méthode de chromatographie en phase gazeuse utilisable à la fois pour la détermination de la pureté du para-xylène et des quantités spécifiques d'impuretés est la méthode ASTM D-3798.

On peut ainsi séparer le produit désiré (*para*-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Le procédé de récupération de *para*-xylène selon l'invention utilisant l'adsorbant décrit selon l'invention présente l'avantage de maximiser la productivité, c'est-à-dire de maximiser le débit de charge à traiter. Ceci est particulièrement vrai dans les conditions opératoires d'unité industrielle d'adsorption de type contre-courant simulé suivantes :

- nombre de lits : 6 à 30,
- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (« stand alone ») et entre 0,7 et 1,4 pour une unité d'adsorption combinée à une unité de cristallisation,
- taux de recyclage (i.e. ratio du débit de recyclage moyen (moyenne des débits de zones pondérée du nombre de lits par zones) sur le débit de charge) compris entre 2,5 et 12, de préférence entre 3,5 et 6.

On pourra sur ce sujet se référer à l'enseignement des brevets US2985589, US5284992 et US5629467. Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US4402832 et US4498991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le *para*-diéthylbenzène (PDEB). La sélectivité des adsorbants selon l'invention pour l'adsorption du *para*-xylène contenu dans des coupes aromatiques en C8 est optimale lorsque leur perte au feu mesurée à 950°C est comprise en général entre 4,0% et 7,7%, et de préférence entre 4,5% et 6,5%, et de manière très préférée entre 4,8% et 6,0% bornes incluses.

Outre l'utilisation précitée de séparation des isomères du xylène, la présente invention concerne également les utilisations des adsorbants décrits ci-dessus comme agents d'adsorption susceptibles de remplacer avantageusement les agents d'adsorption de l'art antérieur pour :

- la séparation d'isomères de toluène substitué tels que nitrotoluène, diéthyltoluène, toluènediamine, et autres,
- la séparation des crésols, et
- la séparation des alcools polyhydriques, tels que les sucres.

L'invention porte enfin sur un procédé de récupération de para-xylène à haute pureté à partir de coupes d'isomères aromatiques à 8 atomes de carbone comprenant les étapes successives suivantes :

a) une étape de mise en contact de la charge avec un lit d'adsorbant comprenant au moins un adsorbant zéolithique tel que défini selon l'une quelconque des revendications 1 à 8 ;
b) une étape de mise en contact du lit d'adsorbant avec un désorbant, de préférence choisi parmi le toluène et le para-diéthylbenzène en phase liquide ou en phase gazeuse.

TECHNIQUES DE CARACTÉRISATION

**Granulométrie des cristaux** :

L'estimation du diamètre moyen en nombre des cristaux de zéolithe X utilisées à l'étape a) et des cristaux de zéolithe X contenue dans les agglomérés est réalisée par observation au microscope électronique à balayage (MEB) ou par observation au microscope électronique en transmission (MET).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié, par exemple le logiciel Smile View de l'éditeur LoGraMi. La précision est de l'ordre de 3%.

**Analyse chimique des adsorbants zéolithiques - rapport Si/Al, teneur pondérales en oxydes et taux d'échange** :

Une analyse chimique élémentaire du produit final obtenu à l'issue des étapes du procédé de l'invention décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids. Dans la présente invention les teneurs en baryum, en strontium, en silicium et en aluminium sont de préférence mesurées par la méthode fluorescence X décrite ci-dessus.

En revanche pour les éléments plus légers (relativement à leur poids atomique, tels que le sodium ou le potassium présents dans l'adsorbant), on préférera pour plus de précision la spectrométrie d'émission atomique avec plasma induit par haute fréquence (ICP-OES pour « Inductively Coupled Plasma-Optical Emission Spectroscopy » selon la terminologie anglo-saxonne) selon la norme UOP 961-12.

L'ICP est une méthode d'analyse par spectrométrie d'émission atomique dont la source est un plasma généré par couplage inductif. Cette méthode est également couramment employée pour déterminer les teneurs en divers éléments tels que le silicium, l'aluminium, le potassium, le sodium, le baryum et le strontium. Dans la présente invention les teneurs en sodium (et éventuellement en potassium pour des teneurs faibles inférieur à 0,5% en poids d'oxyde par rapport à la masse totale d'oxydes) sont de préférence mesurées par la méthode ICP selon la norme UOP 961-12. On obtient dans ce cas pour le sodium une incertitude sur la mesure inférieure à 0,01% pour la teneur en poids de l'oxyde de sodium dans l'adsorbant et pour le potassium une incertitude sur la mesure inférieure à 0,02% pour la teneur en poids de l'oxyde de potassium dans l'adsorbant.

Ces analyses chimiques élémentaires permettent à la fois de vérifier le rapport atomique Si/Al de la zéolithe au sein de l'agglomérat, et de vérifier la qualité de l'échange ionique opéré dans le procédé décrit précédemment. Dans la description de la présente invention, l'incertitude de mesure du ratio atomique Si/Al est de 0,05.

La qualité de l'échange ionique est liée au nombre de mole d'oxyde de sodium ($Na_2O$), restant dans l'adsorbant zéolithique aggloméré après échange. Plus précisément, le taux d'échange par les ions baryum est estimé en évaluant le rapport entre le nombre de moles d'oxyde de baryum, BaO, et le nombre de moles de l'ensemble (BaO + SrO + $Na_2O$ + $K_2O$). De même, le taux d'échange par les ions strontium est estimé en évaluant le rapport entre le nombre de moles d'oxyde de strontium (SrO) et le nombre de moles de l'ensemble (BaO + SrO + $Na_2O$ + $K_2O$).Il est à noter que les teneurs en différents oxydes sont donnés, en pourcentage en poids par rapport au poids total de l'adsorbant anhydre (poids corrigé de la perte au feu).

**Granulométrie des adsorbants zéolithiques** :

La détermination du diamètre moyen en nombre des adsorbants zéolithiques obtenus à l'issus de l'étape d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les adsorbants selon l'invention, avec une précision de l'ordre de 0,01 mm.

**Résistance mécanique des adsorbants zéolithiques** :

La technique de caractérisation de la résistance mécanique représentative de l'écrasement de l'adsorbant au sein d'un lit ou d'un réacteur est la technique de caractérisation de la résistance mécanique en lit, telle que décrite dans la méthode Shell série SMS1471-74 (Shell Method Series SMS1471-74 Détermination of Bulk Crushing Strength of Catalysts. Compression-Sieve Method"), associée à l'appareil "BCS Tester" commercialisé par la société Vinci Technologies. Cette méthode, initialement destinée à la caractérisation de catalyseurs de 3 à 6 mm est basée sur l'utilisation d'un tamis de 425 $\mu$m qui va permettre notamment de séparer les fines créées lors de l'écrasement.

L'utilisation d'un tamis de 425 $\mu$m reste adaptée pour des particules de diamètre supérieur à 1,6 mm, mais doit être adaptée selon la granulométrie des adsorbants que l'on cherche à caractériser. La norme ASTM D7084-04 qui décrit également une méthode de mesure de la résistance à l'écrasement en lit de catalyseurs ("Détermination of Bulk Crush Strength of Catalysts and Catalyst Carriers") définit le passage du tamis à utiliser comme étant égal à la moitié du diamètre des particules de catalyseurs à caractériser.

La méthode prévoit une étape préliminaire de tamisage de l'échantillon de catalyseurs ou adsorbants à caractériser. Si une quantité égale à 10% en poids de l'échantillon passe à travers la grille, un tamis de passage plus petit sera utilisé.

Les adsorbants de la présente invention, généralement sous forme de billes ou d'extrudés, ont en général un diamètre moyen en nombre ou une longueur, i.e. plus grande dimension dans le cas des agglomérés non sphériques, comprise entre 0,4 mm et 2 mm, et en particulier comprise entre 0,4 mm et 0,8 mm et de préférence entre 0,4 mm et 0,65 mm. Par conséquent, un tamis adapté tel que moins de 10% en poids de l'échantillon passe à travers la grille lors d'une étape préalable de tamisage est utilisé à la place du tamis de 425 $\mu$m mentionné dans la méthode Shell standard SMS1471-74.

Le protocole de mesure est le suivant : un échantillon de 20 cm$^3$ d'agglomérats, préalablement tamisé avec le tamis adapté (200 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les adsorbants (utilisation de billes de 2 mm de diamètre pour des particules de forme sphérique de diamètre strictement inférieur à 1,6 mm). Les fines obtenues aux différents paliers de pression sont séparées par tamisage (tamis adapté de 200 $\mu$m) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5% massique de fines cumulées. La résistance mécanique à l'écrasement en lit est typiquement comprise entre quelques centaines de kPa et quelques dizaines de MPa et généralement comprise entre 0,3 MPa et 4 MPa. La précision est de manière classique inférieure à 0,1 MPa.

**Phase non zéolithique des adsorbants zéolithiques** :

Le taux de phase non zéolithique, par exemple liant résiduel non zéolithisé ou toute autre phase amorphe, après zéolithisation est calculé selon l'équation suivante :

$$PNZ = 100 - \Sigma\ (PZ),$$

où PZ représente la somme des quantités des fractions zéolithiques X au sens de l'invention.

La quantité des fractions zéolithiques X est mesurée par analyse par diffraction de rayons X, connue de l'homme du métier sous l'acronyme DRX. Cette analyse est réalisée sur un appareil de la marque Bruker, puis la quantité des fractions zéolithiques X est évaluée au moyen du logiciel TOPAS de la société Bruker. Cette méthode permet également de déterminer la nature des différentes fractions zéolithiques présentes dans l'adsorbant de la présente invention.

**Volume microporeux :**

La cristallinité des agglomérats est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction. Préalablement à l'adsorption, l'adsorbant zéolithique est dégazé entre 300°C et 450°C pendant une durée de 9 heures à 16 heures, sous vide (P < 6,7.10$^{-4}$ Pa). La mesure de l'isotherme d'adsorption d'azote à 77K est ensuite effectuée sur un appareil de type ASAP 2010 M de Micromeritics, en prenant au moins 35 points de mesure à des pressions relatives de rapport PIPa compris entre 0,002 et 1. Le volume microporeux est déterminé selon Dubinin et Raduskevitch à partir de l'isotherme obtenue, en appliquant la norme ISO 15901-3:2007. Le volume microporeux évalué selon Dubinin et Raduskevitch s'exprime en $cm^3$ d'adsorbat liquide par gramme d'adsorbant anhydre. L'incertitude de mesure est de $\pm$ 0,003 $cm^3.g^{-1}$.

**Perte au feu des adsorbants zéolithiques** :

La perte au feu est déterminée en atmosphère oxydante, par calcination de l'échantillon à l'air à une température de 950°C $\pm$ 25°C, comme décrit dans la norme NF EN 196-2 (avril 2006). L'écart type de mesure est inférieur à 0,1%.

**Caractérisation de l'adsorption en phase liquide par perçage** :

La technique utilisée pour caractériser l'adsorption de molécules en phase liquide sur un solide poreux est la technique dite de perçage, décrite par Ruthven dans « *Principles of Adsorption and Adsorption Processes* », Chapitres 8 et 9, John Wiley & Sons, (1984), qui définit la technique des courbes de perçage (« breakthrough curves ») comme l'étude de la réponse à l'injection d'un échelon de constituants adsorbables. L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables. L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

**Exemples**

Les exemples qui suivent illustrent l'invention sans toutefois la limiter de quelque manière que ce soit et dont la portée de protection est précisée par les revendications annexées.

**Méthode générale de préparation d'un adsorbant selon l'invention à base de zéolithique X de ratio molaire Si/Al = 1,25**

On prépare un mélange homogène et on agglomère 800 g de cristaux de zéolithe NaX selon le mode opératoire décrit dans la demande de brevet WO2014090771 (synthèse de l'exemple B) avec 105 g de kaolin (exprimés en équivalent calciné) et 45 g de silice colloïdale vendue sous la dénomination commerciale Klebosol®30 (contenant 30% en poids de $SiO_2$ et 0,5% de $Na_2O$) avec la quantité d'eau qui permet l'extrusion du mélange.

Les extrudés sont séchés, concassés de manière à récupérer des grains dont le diamètre moyen en nombre est égal à 0,5 mm, puis calcinés à 550°C sous courant d'azote pendant 2 heures.

L'aggloméré obtenu (200 g) est placé dans un réacteur en verre muni d'une double enveloppe régulée à une température de 100°C $\pm$ 1°C. On ajoute ensuite 1,5 L d'une solution aqueuse d'hydroxyde de sodium de concentration 2,5 M et on laisse le milieu réactionnel sous agitation pendant une durée de 4 heures.

On procède ensuite au lavage des agglomérés en 3 opérations successives de lavage à l'eau suivi de la vidange du réacteur. On s'assure de l'efficacité du lavage en mesurant le pH final des eaux de lavage compris entre 10,0 et 10,5.

Les cations sodium des agglomérés obtenus sont échangés à 95°C par des ions baryum et strontium. Pour cela, on ajoute au sel de baryum, de formule $BaCl_2$, $2H_2O$, (pur, contenant au maximum 0,2% en poids de chlorure de strontium) différentes quantités de sel de strontium, de formule $SrCl_2$, $6H_2O$, de telle sorte que le pourcentage massique en sel $SrCl_2$ soit égal au pourcentage indiqué dans le Tableau 1 ci-dessous.

Par exemple, on réalise une solution d'échange en mettant en solution 150 g de sel de $BaCl_2$, 2 $H_2O$ avec 1,4 g de sel $SrCl_2$, 6 $H_2O$ dans 1 L d'eau. La quantité en sel de strontium correspond à 0,9% en poids par rapport à la masse totale en sel. Ensuite, 10 g d'agglomérés préparés ci-dessus sont mis en contact avec cette solution afin de réaliser l'échange cationique.

L'échange se fait en 4 étapes. À chaque étape, le rapport volume de solution sur masse de solide est de 25 $mL.g^{-1}$ et l'échange est poursuivi pendant 4 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

La perte au feu mesurée, comme décrit précédemment, est de 5,5% $\pm$ 0,1% pour chaque échantillon. Le taux d'échange en baryum + strontium des adsorbants est calculé à partir des analyses élémentaires par fluorescence X des oxydes de baryum, de strontium et de sodium comme décrit dans les techniques de caractérisation.

Dans l'exemple donné ci-dessus le taux d'échange baryum est de 95,1%, le taux d'échange strontium est de 4,0%.

Les autres exemples sont réalisés en partant de 150 g de sel de baryum auquel on ajoute la quantité de sel $SrCl_2$ permettant d'obtenir les % pondéraux indiqués pour les exemples selon l'invention et les exemples comparatifs.

**Exemple de référence A**

Cet exemple correspond à l'exemple 1 de la demande WO2014090771 et correspond à un adsorbant échangé au baryum seul, avec une très faible teneur en sodium (cf. Tableau 1).

Le taux d'échange au baryum calculé d'après l'analyse en fluorescence X de cet aggloméré est de 99,1 % et la perte au feu de 5,4%.

**Exemple de référence B**

Pour ce second exemple de référence, on refait à l'identique l'exemple 1 de la demande WO2014090771, mais on s'arrête au second échange. Les agglomérats sont engagés dans une réaction d'échange cationique par action d'une solution aqueuse de chlorure de baryum 0,5 M à 95°C en 2 étapes seulement. À chaque étape, le rapport volume de solution sur masse de solide est de 20 mL.g$^{-1}$ et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 heures et enfin activés à 250°C pendant 2 heures sous courant d'azote.

Le taux d'échange au baryum de cet aggloméré est de 92,6 % et la perte au feu de 5,5%.

Les exemples 1 à 5 et l'Exemple Comparatif sont réalisés selon l'exemple de référence A en utilisant des solutions de chlorure de baryum contenant des teneurs croissantes en chlorure de strontium, afin de simuler des solutions de chlorure de baryum contenant du strontium comme L'exemple 5 n'est pas un exemple selon l'invention.

Les détails de chacun des exemples 1 à 5 et de l'Exemple Comparatif sont reproduits dans le Tableau 1 ci-dessous:

-- Tableau 1 --

| *Exemple* | *% SrCl$_2$* | *% BaO* | *% SrO* | *% Na$_2$O* | *Ba/Sr* |
|---|---|---|---|---|---|
| Exemple A | 0 | 37,6 | 0 | < 0,1 | 0 |
| Exemple B | 0 | 34,3 | 0 | 1,1 | 0 |
| Exemple 1 | 0,4 | 35,8 | 0,5 | < 0,2 | 75,8 |
| Exemple 2 | 0,9 | 34,9 | 1,0 | < 0,2 | 35,0 |
| Exemple 3 | 1,7 | 34,8 | 1,7 | < 0,2 | 21,1 |
| Exemple 4 | 2,0 | 34,8 | 2,0 | < 0,2 | 18,4 |
| Exemple 5 | 2,5 | 34,2 | 2,4 | < 0,2 | 15,1 |
| Exemple Comparatif | 4,8 | 33,8 | 3,7 | < 0,2 | 9,7 |

Dans le Tableau 1 ci-dessus :

- % **SrCl$_2$** désigne le pourcentage pondéral de chlorure de strontium hexahydraté dans la solution de chlorure de baryum (comprenant baryum et impuretés) utilisée pour réaliser l'échange cationique ;
- % **BaO** désigne le pourcentage pondéral d'oxyde de baryum par rapport au poids total de l'adsorbant anhydre ;
- % **SrO** désigne le pourcentage pondéral d'oxyde de strontium par rapport au poids total de l'adsorbant anhydre;
- % **Na$_2$O** désigne le pourcentage pondéral d'oxyde de sodium par rapport au poids total de l'adsorbant anhydre;
- **Ba/Sr** désigne le ratio pondéral baryum/strontium dans l'adsorbant anhydre;

**Test de perçage**

Un test de perçage (chromatographie frontale) est ensuite réalisé sur les agglomérés obtenus à l'exemple 1 pour évaluer leur efficacité. La quantité d'adsorbant utilisée pour ce test est d'environ 30 g.

Le mode opératoire pour obtenir les courbes de perçage est le suivant :

- Remplissage de la colonne par le tamis et mise en place dans le banc de test.

- Remplissage par un solvant (toluène) à température ambiante.
- Montée progressive à la température d'adsorption sous flux de solvant (2 cm$^3$.min$^{-1}$).
- Injection de solvant à 2 cm$^3$.min$^{-1}$ lorsque la température d'adsorption est atteinte.
- Permutation solvant/charge pour injecter la charge (2 cm$^3$.min$^{-1}$).
- L'injection de la charge est ensuite maintenue un temps suffisant pour atteindre l'équilibre thermodynamique.
- Collecte de la recette du perçage dans un flacon unique puis analyse de la composition de la recette par CPG.

La pression est suffisante pour que la charge reste en phase liquide, soit 1 MPa. La température d'adsorption est de 175°C. La composition de la charge utilisée pour les tests est la suivante:

- Para-xylène : 18% poids
- Méta-xylène : 18% poids
- Ortho-xylène : 18% poids
- Éthylbenzène : 18% poids
- Para-diéthylbenzène : 18% poids
- Iso-octane : 10% poids (celui-ci est utilisé comme traceur pour l'estimation des volumes non-sélectifs et n'intervient pas dans la séparation)

Les sélectivités binaires des composés deux à deux, notées sélectivités binaires $\alpha_{i/k}$ sont calculées à partir des quantités adsorbées $q_i$ et $q_k$ des composés i et k, ces dernières étant déterminées par bilan matière à partir de l'analyse de la composition de la recette du perçage et de la composition de la charge (charge dans laquelle la fraction

$$\alpha_{i/k} = \frac{q_i y_k}{q_k y_i}$$

massique des composés i et k est $y_i$ et $y_k$) :

L'évaluation du potentiel de ces adsorbants lors de la mise en oeuvre à contre-courant simulé, est faite en se basant sur la théorie de l'équilibre appliquée aux systèmes multi-constituants à sélectivités constantes telle que décrite par Mazotti, Storti et Morbidelli dans « Robust Design of Countercurrent Adsorption Séparation Processes : 2. Multi-component Systems », AIChE Journal, (novembre 1994), Vol. 40, n° 11.

En particulier, on se réfère ici à l'équation 8, qui décrit les conditions à satisfaire sur les débits réduits $m_j$ des 4 sections (j = 1 à j = 4) d'une unité de séparation à contre-courant telle que schématisée en Figure 1 de l'article cité pour obtenir une séparation complète.

$$\begin{aligned}
&\text{Section 1:} && K_{ss} < m_1\delta_1 < +\infty \\
&\text{Section 2:} && K_{wk} < m_2\delta_2 < K_{sk} \\
&\text{Section 3:} && K_{wk} < m_3\delta_3 < K_{sk} \\
&\text{Section 4:} && -\frac{\epsilon_p\delta_4}{\sigma(1-\epsilon_p)} < m_4\delta_4 < K_{ww}.
\end{aligned} \qquad (8)$$

Cette équation 8 fait référence aux adsorptivités $K_i$ des différents constituants, ainsi qu'au paramètre $\delta_j$ de chaque section j défini par l'équation 7 :

$$\delta_j = \sum_{l=1}^{NC} K_l y_l^j \quad (j = 1, \ldots, 4), \qquad (7)$$

Il faut noter ici que par définition la sélectivité binaire $\alpha_{i/k}$ entre les composés i et k est égal au rapport des adsorptivités $K_i/K_k$.

Le débit réduit « m » de chaque section de l'unité est défini comme étant le rapport du débit de la phase liquide sur le débit de la phase adsorbée. L'équation 8 indique quels sont les débits réduits limites pour chaque section. Dans une unité de séparation à contre-courant à 4 sections, le débit de charge correspond à la différence entre le débit réduit en zone 3 et le débit réduit en zone 2.

Par conséquent, lorsque l'on veut évaluer la productivité maximale qui peut être atteinte avec un adsorbant donné, on cherche à évaluer la quantité maximale de charge que l'on pourra traiter, c'est-à-dire à évaluer la différence entre le débit réduit maximal en zone 3 et le débit réduit minimal en zone 2.

On pourra comparer les performances en terme de productivité maximale de deux adsorbants en comparant leur débit réduit maximal de charge déterminé à partir des débits réduits des zones 2 et 3, respectivement $m_2$ et $m_3$, selon la relation :

$$\max(m_{Charge}) = \max(m_3) - \min(m_2).$$

Si on considère un système à sélectivités constantes, la composition de la phase liquide qui donne la contrainte la plus forte en zone 2 et en zone 3 est la composition de la phase liquide au point d'injection de la charge dans l'unité. En effet, à partir de ce point la concentration en para-xylène, qui est le composé le plus adsorbé, augmente dans le sens de circulation du solide en zone 2, et diminue dans le sens de circulation du liquide en zone 3. On peut approximer la composition de ce point à la composition de la charge à traiter, et c'est cette composition qui sera utilisée pour évaluer le terme $\delta_2$ et $\delta_3$ de l'équation 8. Les termes $\delta_2$ et $\delta_3$ étant définis par l'équation 7 mentionnée ci-dessus.

Pour chaque adsorbant, ce débit réduit $\max(m_{Charge})$ est calculé à partir des valeurs de sélectivités binaires mesurées expérimentalement. Le Tableau 2, permet de comparer le débit réduit maximal de charge « $\max(m_{Charge})$ », pour chacun des adsorbants testés. Le débit réduit maximal de charge « $\max(m_{Charge})$ » est représentatif de la productivité, plus sa valeur est élevée, meilleure est la productivité.

-- Tableau 2 --

|  | $\alpha_{PX/MOX}$ | $\alpha_{PX/EB}$ | $max(m_{Charge})$ |
|---|---|---|---|
| Exemple A | 3,10 | 2,30 | 1,17 |
| Exemple B | 2,92 | 2,23 | 1,11 |
| Exemple 2 | 3,26 | 2,26 | 1,18 |
| Exemple Comparatif | 2,85 | 2,19 | 1,08 |

On s'aperçoit que le débit réduit $\max(m_{Charge})$ reste sensiblement le même qu'il s'agisse d'un adsorbant dont l'échange ionique a été réalisé avec une solution de chlorure de baryum « pure » (i.e. sans impureté strontium) (Exemple A) ou d'un adsorbant préparé à partir d'une solution de chlorure de baryum contenant également du chlorure de strontium (Exemple 2).

En revanche lorsque le chlorure de baryum contient du sodium à des teneurs comparables au strontium (en raison de l'échange partiel au baryum), on remarque que la productivité $\max(m_{Charge})$ est réduite. Cette observation confirme effectivement, outre l'effet sur la sélectivité, l'intérêt de l'échange au baryum pour la séparation des xylènes, ce qui est parfaitement connu de l'homme du métier.

De même, il a été observé que lorsque la teneur en impuretés strontium dans la solution de chlorure de baryum est trop importante, en particulier supérieure à 4% (cf. Exemple comparatif, teneur en chlorure de strontium > 4,8%), la productivité $\max(m_{Charge})$ chute drastiquement.

Ces exemples confirment en tous points l'objet de la présente invention et permettent de mettre en évidence qu'il est tout à fait possible d'envisager la présence d'ions strontium dans des adsorbants échangés au baryum utilisables pour la séparation des xylènes, sans pour autant affecter la productivité en para-xylène. Par ailleurs, une amélioration de sélectivité pour le para-xylène est obtenue.

**Revendications**

1. Adsorbant à base de cristaux de zéolithe(s) agglomérés, ledit aggloméré comprenant :

- au moins des cristaux de zéolithe(s) FAU, de ratio molaire Si/Al compris entre 1,00 et 1,50, bornes incluses,
- une teneur pondérale en ions baryum ($Ba^{2+}$), exprimée en poids d'oxyde de baryum (BaO), strictement supérieure à 30%, de préférence strictement supérieure à 33%, de préférence encore une teneur comprise entre 34% et 42%, bornes incluses, et de manière tout à fait préférée comprise entre 34% et 40%, bornes incluses, par rapport au poids total de l'adsorbant,
- une teneur pondérale en ions strontium ($Sr^{2+}$), exprimée en poids d'oxyde de strontium (SrO), strictement supérieure à 0,1 % et strictement inférieure à 3%, de préférence comprise entre 0,15% et 2,9%, bornes incluses, de préférence encore comprise entre 0,15% et 2,5% et de manière tout à fait préférée comprise entre 0,15% et 2,4%, bornes incluses, par rapport au poids total de l'adsorbant ; et

dans lequel l'adsorbant présente un ratio pondéral baryum/strontium compris entre 16:1 et 400:1.

2.   Adsorbant selon la revendication 1, dans lequel « zéolithe(s) FAU de type X» désigne les zéolithes dont le ratio atomique Si/Al est compris entre 1,00 et 1,50 bornes incluses, de préférence entre 1,00 et 1,45, de préférence encore entre 1,05 et 1,45, bornes incluses et de manière encore plus préférée entre 1,10 et 1,45 bornes incluses.

3.   Adsorbant selon l'une des revendications 1 ou 2, présentant un rapport atomique Si/Al compris entre 1,00 et 2,00 de préférence entre 1,00 et 1,80 bornes incluses, de préférence encore entre 1,15 et 1,80, bornes incluses et de manière encore plus préférée entre 1,15 et 1,60, bornes incluses.

4.   Adsorbant selon l'une quelconque des revendications précédentes, dans lequel la teneur pondérale totale en ions alcalins ou alcalino-terreux autres que $Ba^{2+}$ et $Sr^{2+}$, exprimée en poids d'oxydes d'alcalins ou d'alcalino-terreux respectivement, est inférieure à 5% et de préférence est comprise entre 0% et 2% et avantageusement entre 0% et 1 %, bornes incluses, par rapport au poids total de l'adsorbant.

5.   Adsorbant selon l'une quelconque des revendications précédentes, dans lequel la teneur pondérale en ions sodium ($Na^+$), exprimée en poids d'oxyde de sodium ($Na_2O$), est strictement inférieure à 0,3%, de préférence strictement inférieure à 0,2%, par rapport au poids total de l'adsorbant.

6.   Adsorbant selon l'une quelconque des revendications précédentes, dans lequel la teneur en ions potassium ($K^+$), exprimée en poids d'oxyde de potassium ($K_2O$), est inférieure à 9%, de préférence inférieure à 8%, mieux encore inférieure à 5%, et de préférence encore comprise entre 0% et 2%, avantageusement entre 0% et 1%, bornes incluses, par rapport au poids total de l'adsorbant.

7.   Adsorbant selon l'une quelconque des revendications précédentes, dans lequel le ratio pondéral baryum/strontium dans l'adsorbant est compris entre 16:1 et 300:1 , de préférence encore compris entre 16:1 et 200:1 , bornes incluses.

8.   Adsorbant selon l'une quelconque des revendications précédentes, dans lequel la teneur pondérale en phase non zéolithique (PNZ) est inférieure à 15%, de préférence inférieure à 10%, de préférence encore inférieure à 5%, et typiquement comprise entre 0 et 15% de préférence comprise entre 0 et 10%, de préférence encore comprise entre 0 et 5% bornes non comprises, de manière tout à fait préférée comprise entre 0,1% et 5%, mieux encore entre 0,5% et 5% et de manière tout particulièrement préférée entre 2% et 5%, bornes non comprises, par rapport au poids total de l'adsorbant anhydre.

9.   Utilisation d'un adsorbant selon l'une quelconque des revendications 1 à 8, pour la production en phase liquide ou phase gaz de para-xylène très pur à partir d'une charge d'hydrocarbures aromatiques contenant des isomères à 8 atomes de carbone.

10.  Procédé de récupération de para-xylène à haute pureté à partir de coupes d'isomères aromatiques à 8 atomes de carbone comprenant les étapes successives suivantes :

a) une étape de mise en contact de la charge avec un lit d'adsorbant comprenant au moins un adsorbant zéolithique tel que défini selon l'une quelconque des revendications 1 à 8 ;
b) une étape de mise en contact du lit d'adsorbant avec un désorbant, de préférence choisi parmi le toluène et le para-diéthylbenzène en phase liquide ou en phase gazeuse.

**Patentansprüche**

1.   Adsorptionsmittel auf der Grundlage von agglomerierten Zeolithkristallen, wobei das Agglomerat Folgendes umfasst:

- mindestens FAU-Zeolithkristalle mit einem Molverhältnis Si/Al im Bereich zwischen 1,00 und 1,50, Grenzen eingeschlossen,
- einen Gewichtsanteil von Bariumionen ($Ba^{2+}$), exprimiert in Bariumoxidgewicht (BaO), strikt über 30 %, vorzugsweise strikt über 33 %, noch bevorzugter einen Anteil im Bereich zwischen 34 % und 42 %, Grenzen eingeschlossen, und am meisten bevorzugt im Bereich zwischen 34 % und 40 %, Grenzen eingeschlossen, in Bezug auf das Gesamtgewicht des Absorptionsmittels,
- einen Gewichtsanteil von Strontiumionen ($Sr^{2+}$), exprimiert in Strontiumoxidgewicht (SrO), strikt über 0,1 % und strikt unter 3 %, vorzugsweise im Bereich zwischen 0,15 % und 2,9 %, Grenzen eingeschlossen, noch bevorzugter im Bereich zwischen 0,15 % und 2,5 % und am meisten bevorzugt im Bereich zwischen 0,15 % und 2,4 %, Grenzen eingeschlossen, mit Bezug auf das Gesamtgewicht des Absorptionsmittels; und

wobei das Adsorptionsmittel ein Gewichtsverhältnis Barium/Strontium aufweist, das im Bereich zwischen 16:1 und 400:1 liegt.

2. Adsorptionsmittel nach Anspruch 1, wobei "FAU-Zeolith(en) vom Typ X" Zeolithen bezeichnet, deren Atomverhältnis Si/Al im Bereich zwischen 1,00 und 1,50, Grenzen eingeschlossen, liegt, vorzugsweise zwischen 1,00 und 1,45, noch bevorzugter zwischen 1,05 und 1,45, Grenzen eingeschlossen, und noch weiter bevorzugt zwischen 1,10 und 1,45, Grenzen eingeschlossen, liegt.

3. Adsorptionsmittel nach einem der Ansprüche 1 oder 2, aufweisend ein Atomverhältnis Si/Al, das zwischen 1,00 und 2,00 liegt, vorzugsweise zwischen 1,00 und 1,80, Grenzen eingeschlossen, noch bevorzugter zwischen 1,15 und 1,80, Grenzen eingeschlossen, und noch weiter bevorzugt zwischen 1,15 und 1,60, Grenzen eingeschlossen.

4. Adsorptionsmittel nach einem der vorhergehenden Ansprüche, wobei der gesamte Gewichtsanteil an alkalischen oder erdalkalischen Ionen außer $Ba^{2+}$ und $Sr^{2+}$, exprimiert in Alkali- bzw. Erdalkalioxidgewicht, kleiner als 5 % ist und vorzugsweise im Bereich zwischen 0 % und 2 % und vorteilhafterweise zwischen 0 % und 1 %, Grenzen eingeschlossen, mit Bezug auf das Gesamtgewicht des Absorptionsmittels liegt.

5. Adsorptionsmittel nach einem der vorhergehenden Ansprüche, wobei der Gewichtsanteil an Natriumionen ($Na^+$), exprimiert in Natriumoxidgewicht ($Na_2O$), strikt unter 3 %, vorzugsweise strikt unter 0,2 % in Bezug auf das Gesamtgewicht des Absorptionsmittels ist.

6. Adsorptionsmittel nach einem der vorhergehenden Ansprüche, wobei der Gehalt an Kaliumionen ($K^+$), exprimiert in Kaliumoxidgewicht ($K_2O$), kleiner als 9 %, vorzugsweise kleiner als 8 %, noch besser kleiner als 5 % ist und noch bevorzugter im Bereich zwischen 0 % und 2 %, vorteilhafterweise zwischen 0 % und 1 %, Grenzen eingeschlossen, in Bezug auf das Gesamtgewicht des Absorptionsmittels liegt.

7. Adsorptionsmittel nach einem der vorhergehenden Ansprüche, wobei das Gewichtsverhältnis Barium/Strontium im Absorptionsmittel im Bereich zwischen 16:1 und 300:1 liegt, noch bevorzugter im Bereich zwischen 16:1 und 200:1, Grenzen eingeschlossen, liegt.

8. Adsorptionsmittel nach einem der vorhergehenden Ansprüche, wobei der Gewichtsanteil in der nicht zeolithischen Phase (PNZ) kleiner als 15 %, vorzugsweise kleiner als 10 %, noch bevorzugter kleiner als 5 % ist und typischerweise im Bereich zwischen 0 und 15 %, vorzugsweise im Bereich zwischen 0 und 10 %, noch bevorzugter im Bereich zwischen 0 und 5 %, Grenzen nicht eingeschlossen, am meisten bevorzugt im Bereich zwischen 0,1 % und 5 % liegt, noch besser zwischen 0,5 % und 5 % und auf ganz besonders bevorzugte Weise zwischen 2 % und 5 %, Grenzen nicht eingeschlossen, in Bezug auf das Gesamtgewicht des wasserfreien Absorptionsmittels liegt.

9. Verwendung eines Adsorptionsmittels nach einem der Ansprüche 1 bis 8 für die Herstellung in flüssiger Phase oder gasförmiger Phase von sehr reinem Paraxylol ausgehend von einer Charge aromatischer Kohlenwasserstoffe, enthaltend Isomere mit 8 Kohlenstoffatomen.

10. Verfahren zur Rückgewinnung von Paraxylol mit hoher Reinheit ausgehend von Schnitten von aromatischen Isomeren mit 8 Kohlenstoffatomen, umfassend die folgenden sukzessiven Schritte:

a) einen Schritt des In-Kontakt-Bringens der Charge mit einem Absorptionsmittelbett, umfassend mindestens ein zeolithisches Absorptionsmittel wie nach einem der Ansprüche 1 bis 8 definiert;
b) einen Schritt des In-Kontakt-Bringens des Absorptionsmittelbetts mit einem Desorptionsmittel, vorzugsweise ausgewählt aus Toluol und para-Diethylbenzol in flüssiger Phase oder in gasförmiger Phase.

**Claims**

1. Adsorbent based on agglomerated crystals of zeolite(s), said agglomerate comprising:

   - at least crystals of FAU zeolite(s), with an Si/Al molar ratio of between 1.00 and 1.50, limits included;
   - a weight content of barium ions ($Ba^{2+}$), expressed by weight of barium oxide (BaO), strictly greater than 30%, preferably strictly greater than 33%, more preferably a content of between 34% and 42%, limits included, and entirely preferably of between 34% and 40%, limits included, relative to the total weight of the adsorbent; and
   - a weight content of strontium ions ($Sr^{2+}$), expressed by weight of strontium oxide (SrO), strictly greater than 0.1% and strictly less than 3%, preferably of between 0.15% and 2.9%, limits included, more preferably of between 0.15% and 2.5% and entirely preferably of between 0.15% and 2.4%, limits included, relative to the total weight of the adsorbent, and

   the adsorbent has a barium/strontium weight ratio between 16:1 and 400:1.

2. Adsorbent according to claim 1, in which the term "FAU zeolite(s) of type X" denotes zeolites of which the Si/Al atomic ratio is between 1.00 and 1.50, limits included, preferably between 1.00 and 1.45, more preferably between 1.05 and 1.45, limits included, and even more preferably between 1.10 and 1.45, limits included.

3. Adsorbent according to either claims 1 and 2, having an Si/Al atomic ratio of between 1.00 and 2.00, preferably between 1.00 and 1.80, limits included, more preferably between 1.15 and 1.80, limits included, and even more preferably between 1.15 and 1.60, limits included.

4. Adsorbent according to any one of the preceding claims, in which the total weight content of alkali or alkaline-earth metal ions other than $Ba^{2+}$ and $Sr^{2+}$, expressed by weight of alkali or alkaline-earth metal oxides respectively, is less than 5%, and is preferably between 0% and 2% and advantageously between 0% and 1%, limits included, relative to the total weight of the adsorbent.

5. Adsorbent according to any one of the preceding claims, in which the weight content of sodium ions ($Na^+$), expressed by weight of sodium oxide ($Na_2O$), is strictly less than 0.3%, preferably strictly less than 0.2%, relative to the total weight of the adsorbent.

6. Adsorbent according to any one of the preceding claims, in which the content of potassium ions ($K^+$), expressed by weight of potassium oxide ($K_2O$), is less than 9%, preferably less than 8%, even better still less than 5%, and more preferably of between 0% and 2%, advantageously between 0% and 1%, limits included, relative to the total weight of the adsorbent.

7. Adsorbent according to any one of the preceding claims, in which the barium/strontium weight ratio in the adsorbent is between 16:1 and 300:1, more preferably between 16:1 and 200:1, limits included.

8. Adsorbent according to any one of the preceding claims, in which the weight content of non-zeolite phase (NZP) is less than 15%, preferably less than 10%, more preferably less than 5% and is typically between 0% and 15%, preferably between 0% and 10%, more preferably between 0% and 5%, limits not included, entirely preferably between 0.1% and 5%, even better still between 0.5% and 5% and most particularly preferably between 2% and 5%, limits not included, relative to the total weight of the anhydrous adsorbent.

9. Use of an adsorbent according to any one of claims 1 to 8, for the liquid-phase or gas-based production of very pure para-xylene from a feedstock of aromatic hydrocarbons containing isomers comprising 8 carbon atoms.

10. Process for recovering high-purity para-xylene from fractions of aromatic isomers comprising 8 carbon atoms, comprising the following successive steps:

    a) bringing a feedstock into contact with a bed of adsorbent comprising at least one zeolite adsorbent as defined according to any one of claims 1 to 8,
    b) bringing the bed of adsorbent into contact with a desorbent, preferably chosen from toluene and para-diethylbenzene, in liquid phase or in gas phase.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2681066
- US 3558732 A
- US 4255607 A
- FR 2329623
- US 3997620 A
- US 7785563 B

- WO 2014090771 A
- US 2985589 A
- US 5284992 A
- US 5629467 A
- US 4402832 A
- US 4498991 A

**Littérature non-brevet citée dans la description**

- **MAZOTTI ; STORTI ; MORBIDELLI.** Robust Design of Countercurrent Adsorption Séparation Processes : 2. Multicomponent Systems. *AIChE Journal,* Novembre 1994, vol. 40 (11